(19) Europäisches Patentamt
European Patent Office
Office européen des brevets

(11) EP 0 703 776 B1

(12) **FASCICULE DE BREVET EUROPEEN**

(45) Date de publication et mention de la délivrance du brevet: **23.04.1997 Bulletin 1997/17**

(21) Numéro de dépôt: **94919718.0**

(22) Date de dépôt: **16.06.1994**

(51) Int. Cl.$^6$: **A61K 7/48**, A61K 7/06, A61K 7/135, C07K 5/06

(86) Numéro de dépôt international: **PCT/FR94/00721**

(87) Numéro de publication internationale: **WO 95/00115 (05.01.1995 Gazette 1995/02)**

(54) **UTILISATION D'UN PEPTIDE AYANT UN GROUPEMENT LYSINE ET UN GROUPEMENT ALANINE EN POSITION TERMINALE POUR LA PREPARATION D'UNE COMPOSITION DEPIGMENTANTE ET COMPOSITION DEPIGMENTANTE EN COMPORTANT APPLICATION**

VERWENDUNG EINES PEPTIDS MIT EIN LYSINE UND EIN ENDSTÄNDIGEN ALANINE FÜR EIN ENTFÄRBUNGSZUSAMMENSETZUNG SOWIE IHRE VERWENDUNG

UTILIZATION OF A PEPTIDE HAVING A LYSIN GROUP AND AN ALANINE GROUP IN THE TERMINAL POSITION FOR THE PREPARATION OF A DEPIGMENTING COMPOSITION, AND DEPIGMENTING COMPOSITION THUS OBTAINED

(84) Etats contractants désignés:
**BE CH DE ES FR GB IT LI**

(30) Priorité: **17.06.1993 FR 9307319**

(43) Date de publication de la demande:
**03.04.1996 Bulletin 1996/14**

(73) Titulaire: **PARFUMS CHRISTIAN DIOR**
**75008 Paris (FR)**

(72) Inventeurs:
- **RENIMEL, Isabelle**
  **F-45470 Trainou (FR)**
- **ANDRE, Patrice**
  **F-45170 Neuville-aux-Bois (FR)**
- **REDZINIAK, Gérard**
  **F-45590 Saint-Cyr-en-Val (FR)**

(74) Mandataire: **Portal, Gérard et al**
**Cabinet Beau de Loménie**
**158, rue de l'Université**
**75340 Paris Cédex 07 (FR)**

(56) Documents cités:
**EP-A- 0 413 528**
**WO-A-88/05654**
**WO-A-89/12441**
**WO-A-91/17173**
**FR-A- 2 609 393**

- **DATABASE WPI Week 9311, Derwent Publications Ltd., London, GB; AN 93-088578 & JP,A,5 032 533 (KYOWA HAKKO KOGYO KK) 9 Février 1993**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 703 776 B1

## Description

La présente invention concerne essentiellement l'utilisation d'un peptide ayant un groupement lysine et un groupement alanine en position terminale pour la préparation d'une composition dépigmentante et une composition dépigmentante en comportant application.

Certains peptides particuliers, notamment des peptides de soie, ont déjà été décrits comme pouvant être utilisés, dans les formules cosmétiques, pour inhiber la mélanogénèse (JP 57-04 910).

Il a également été décrit que des dérivés peptidiques de l'acide kojique pouvaient être incorporés dans des compositions dépigmentantes pour la peau (JP 04-187 618).

Or, au cours de recherches intensives faites par les les auteurs de la présente invention, il a été découvert de manière totalement inattendue que les peptides comportant, en extrémité de chaîne, un groupement lysine (Lys) et, à l'autre extrémité de chaîne, un groupement alanine (Ala), présentaient une activité dépigmentante.

Ainsi, la présente invention a pour but principal de fournir une nouvelle classe de composés ayant une bonne activité dépigmentante, ainsi que de nouvelles formulations de compositions cosmétiques ou pharmaceutiques, notamment dermatologiques ayant une activité dépigmentante. L'invention concerne aussi de nouvelles méthodes de dépigmentation comprenant l'application de ces composés ou compositions.

Ce but a été résolu selon la présente invention par la découverte que des peptides comprenant, en extrémité de chaîne, un groupement lysine (Lys) et, à l'autre extrémité de chaîne, un groupement alanine (Ala), présentaient une bonne activité dépigmentante. De préférence, le groupement lysine est situé en position N-terminale, et le groupement alanine en position C-terminale.

Ainsi, selon un premier aspect, la présente invention concerne l'utilisation d'un peptide constitué d'une chaîne peptidique comportant à l'une de ses deux extrémités un groupement lysine (Lys), et à son autre extrémité un groupement alanine (Ala), pour la préparation d'une composition dépigmentante destinée en particulier à atténuer ou à supprimer les taches mélaniques de la peau, telles que les taches dites de sénescence, ou à éclaircir la chevelure.

Avantageusement, le nombre d'acides aminés formant ladite chaîne est compris entre 2 et 10 inclus.

Suivant un mode de réalisation préféré de l'invention, le groupement lysine précité est situé en position dite N-terminale, et le groupement alanine précité est situé en position dite C-terminale.

Ainsi, conformément à la représentation habituellement utilisée par l'homme de l'art pour les chaînes peptidiques, les peptides du mode de réalisation précité de l'invention peuvent être représentés par la formule :

Lys..........Ala (SEQ ID NO: 1)

dans laquelle Lys représente un groupement lysine en position N-terminale, Ala représente un groupement alanine en position C-terminale, et les points de suspension représentent les groupements d'acides aminés intermédiaires.

Suivant un mode de réalisation particulièrement avantageux un tel peptide est le dipeptide Lys-Ala, de formule développée :

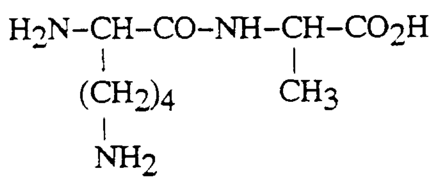

Un autre peptide particulièrement avantageux est l'hexapeptide de formule suivante :

Lys-Cys-Thr-Cys-Cys-Ala. (SEQ ID NO: 2)

dans laquelle notamment Cys représente un groupement cystéine et Thr représente un groupement thréonine.

Il est à noter que le dipeptide Lys-Ala (SEQ ID NO: 1) ou l'hexapeptide (SEQ ID NO: 2) ci-dessus sont commercialement disponibles, notamment chez Sigma, France.

Avantageusement, le peptide selon l'invention précité est utilisé à une concentration comprise entre 0,01 % et 5 % en poids, par rapport au poids total de la composition.

Selon une variante avantageuse de l'invention, le peptide précédemment défini est incorporé au moins en partie dans des vecteurs de substances actives tels qu'une phase lamellaire lipidique hydratée ou des liposomes. Dans ce cas, l'incorporation dans ces vecteurs, en particulier dans ladite phase lamellaire ou les liposomes, peut être réalisée selon des procédés bien connus de l'homme de l'art, relatifs à l'incorporation de peptides dans ces vecteurs.

Par exemple, pour incorporer dans des liposomes les peptides définis dans la présente invention, on peut, tout d'abord, selon la méthode décrite dans le document US-A-4 508 703, préparer une poudre lipidique à base de phospholipides, tels que la lécithine de soja, et, éventuellement, d'un stérol, tel que le cholestérol ou le sitostérol. Cette poudre lipidique est ensuite dispersée dans une solution aqueuse contenant ledit peptide, sous agitation. Il se forme alors

une suspension de liposomes incorporant, au moins en partie, le peptide.

Avantageusement, cette suspension de liposomes peut être homogénéisée, suivant une technique connue de l'homme de l'art, par exemple soit au moyen d'ultrasons, ou bien, suivant un procédé plus particulièrement approprié à une production à l'échelle industrielle, tel qu'une homogénéisation sous pression, comme celui décrit dans le brevet US-A-4 621 023.

Naturellement, le peptide de l'invention, utilisé comme agent dépigmentant, peut être combiné à diverses autres substances actives comme cela est bien compréhensible à un homme de l'art.

Selon un deuxième aspect, la présente invention concerne également une composition cosmétique ou pharmaceutique, notamment dermatologique, à activité dépigmentante, caractérisée en ce qu'elle comprend un peptide tel que précédemment défini, ledit peptide étant incorporé dans un excipient cosmétiquement ou pharmaceutiquement acceptable.

Ce peptide peut également être utilisé à la concentration précédemment définie exprimée en poids sur la base du poids total de la composition.

Conformément à une variante avantageuse de l'invention, le peptide précédemment défini est incorporé au moins en partie dans un vecteur de substance active, comme cela a été exposé ci-dessus.

Suivant un mode avantageux de réalisation de l'invention, la composition précitée est formulée en vue d'une application topique sur la peau, notamment celle des mains ou du visage, ou sur le cuir chevelu. Elle peut se présenter par exemple sous la forme d'un gel, d'une crème, d'un lait ou d'une lotion.

L'invention concerne encore, selon un troisième aspect, un procédé cosmétique de dépigmentation de la peau ou des cheveux, caractérisé en ce qu'il comprend l'application sur les zones de la peau devant être dépigmentées ou sur le cuir chevelu, d'une quantité efficace d'un peptide tel que précédemment défini, incorporé dans une composition cosmétique.

L'activité dépigmentante des peptides selon l'invention sera démontrée par les essais suivants en relation avec les figures annexées.

Dans les figures :

- la figure 1 représente en ordonnées le pourcentage d'inhibition de la tyrosinase par le peptide testé, Lys-Ala (SEQ ID NO: 1), en fonction de la concentration du peptide en mol/l, en abscisses. A chaque concentration, les résultats sont donnés pour trois temps différents, T = 0 min, T = 5 min et T = 15 min, de mise en présence de la tyrosinase avec le peptide Lys-Ala ; et
- la figure 2 représente aussi un histogramme représentant en ordonnées le pourcentage d'inhibition de la tyrosinase par l'hexapeptide Lys-Cys-Thr-Cys-Cys-Ala (SEQ ID NO: 2), en fonction de la concentration du peptide en mol/l, en abcsisses. A chaque concentration, les résultats sont également donnés pour trois temps différents, T = 0 min, T = 5 min et T = 15 min, de mise en présence de la tyrosinase avec l'hexapeptide.

<u>Exemple 1</u>

<u>Activité dépigmentante du peptide Lys-Ala (SEQ ID NO: 1)</u>

L'activité dépigmentante est déterminée de manière connue en soi par un effet d'inhibition de la tyrosinase qui est une enzyme responsable de la synthèse de mélanine, pigment brun plus ou moins foncé, contribuant à la coloration de l'épiderme. A ce propos, il est rappelé que la L-tyrosine, en présence de tyrosinase et d'oxygène, s'oxyde en L-dopa, qui s'oxyde à son tour en dopaquinone, également en présence de tyrosinase et d'oxygène. La dopaquinone est un précurseur de la mélanine. Il est à noter que la tyrosinase est une enzyme tétramérique contenant du cuivre et qui est reconnue comme comprenant deux sites actifs pour les composés aromatiques, par exemple la L-tyrosine et de L-dopa, tout en possédant également deux sites de régulation, l'un pour l'oxygène et l'autre pour le cuivre.

Ainsi, on comprend que lorsque la tyrosinase est inhibée, la production de mélanine est réduite, voire supprimée. De ce fait, lorsque ce phénomène se produit dans la peau, les cellules pigmentées de la couche superficielle de l'épiderme, qui sont éliminées par la desquamation naturelle, sont progressivement remplacées par des cellules ne contenant pas de mélanine, et donc non pigmentées, d'où l'effet dépigmentant.

Pour la mise en évidence de l'activité dépigmentante précitée, on opère comme suit. Le peptide Lys-Ala (SEQ ID NO: 1) disponible dans le commerce, en particulier chez Sigma, France, est mis en solution de manière extemporanée dans un tampon phosphate 0,02 M (pH 6,9). On prépare tout d'abord une solution mère ayant une concentration 5 mM en Lys-Ala, qui peut ensuite être diluée extemporanément pour fournir une gamme de 5-1-0,5-0,05-0,005 mM.

D'autre part, on prépare également une solution de tyrosinase de champignon dans le même tampon phosphate 0,02 M (pH 6,9) à 1 mg/ml d'enzyme (2 000-4 000 unités/milligramme) que l'on conserve à -20°C jusqu'à son utilisation.

On prépare également une solution de substrats contenant à la fois la L-tyrosine et la L-dopa, chacune à la concentration de 1 mM, dans le même tampon phosphate, que l'on conserve à l'abri de la lumière.

Les essais de détermination de l'activité dépigmentante sont réalisés de la manière suivante :

La méthode de détermination de l'activité dépigmentante est basée sur la méthode de dosage décrite dans le livre "Worthington Assay Procedure", Worthington System Inc., Freehold, New Jersey 07728, USA, ainsi que sur la méthode de Pomreantz, J. Biol. Chem. 241, 161 (1966).

La méthode décrite est cependant modifiée de la manière suivante :

- la solution de substrat est un mélange de L-tyrosine et de L-dopa à concentration égale, au lieu de l'emploi de la tyrosine seule dans la méthode décrite dans la littérature ci-dessus ;
- par ailleurs, on suit la cinétique de formation de dopaquinone avec un spectrophotomètre, à la longueur d'onde de 480 nm, au lieu de suivre la disparition de tyrosine à la longueur d'onde appropriée.

On réalise plusieurs séries d'essais. Pour chaque série d'essais, on mélange, en début d'expérience, au temps dit $T_0$ la solution de peptide et la solution de tyrosinase.

Dans une première série d'essais, on mélange également au temps $T_0$ la solution de substrats contenant à la fois la L-tyrosine et la L-dopa. Les résultats obtenus avec cette série d'essais au temps $T_0$ sont rapportés au tableau I pour diverses concentrations en peptide.

Dans une deuxième série d'essais, on ajoute la solution de substrats au temps T = 5 min, soit 5 min après le temps $T_0$, au mélange initial des solutions de peptide et de tyrosinase. Pour cette deuxième série d'essais, on obtient les résultats pour diverses concentrations de peptide sous la mention T 5 min au tableau I.

On effectue une troisième série d'essais en ajoutant la solution de substrats 15 min après le temps $T_0$, au mélange initial des solutions de peptide et de tyrosinase. On obtient des résultats d'essais sous la mention T 15 min au tableau I à diverses concentrations de peptide.

Pour chaque essai de chaque série d'essais précédente, à partir du moment où on a mélangé la solution de substrat avec la solution de peptide et la solution de tyrosinase, on suit pendant environ 2 min la cinétique de formation de dopaquinone, qui est le produit d'oxydation de la L-tyrosine et de la L-dopa en présence de la tyrosinase. Cette cinétique est suivie en mesurant la densité optique du mélange des trois solutions au moyen d'un spectrophotomètre réglé à une valeur de longueur d'onde d'absorption égale à 480 nm.

Parallèlement, on effectue également un essai témoin sans ajout de peptide pour déterminer la densité optique correspondant à 100 % d'activité enzymatique, c'est-à-dire sans aucune inhibition de la tyrosinase.

Le spectrophotomètre est relié à des moyens de calcul, par exemple un ordinateur ou micro-ordinateur contenant un logiciel calculant, d'une part, la valeur de la pente de la partie linéaire ascendante de la courbe de cinétique de formation de la dopaquinone, respectivement pour le témoin et pour chaque essai de chaque série d'essais avec l'agent inhibiteur de l'invention, à savoir ici le peptide, et déterminant le pourcentage d'inhibition selon la formule suivante :

$$\text{pourcentage d'inhibition} = \frac{\text{pente témoin - pente d'essai}}{\text{pente témoin}} \times 100$$

Il est à noter que tous les essais sont effectués 5 fois pour chaque concentration dans chaque série d'essais afin d'obtenir des valeurs moyennes.

Les pourcentages d'inhibition moyens sont rapportés au tableau I.

A la figure 1, on a représenté en ordonnées le pourcentage d'inhibition de la tyrosinase par le dipeptide testé Lys-Ala (SEQ ID NO: 1), en fonction de sa concentration en mol/l, en abscisse.

## TABLEAU I

Activité dépigmentante de Lys–Ala (SEQ ID NO: 1)

| Conc. Lys–Ala / Temps | $5.10^{-6}$ M | $5.10^{-5}$ M | $5.10^{-4}$ M | $1.10^{-3}$ M | $5.10^{-3}$ M |
|---|---|---|---|---|---|
| T 0 | 6 | 8 | 15 | 35 | 56 |
| T 5 min | 8 | 11 | 30 | 59 | 72 |
| T 15 min | 15 | 22 | 67 | 78 | 83 |

Il ressort du tableau I et de la figure 1 annexée que le peptide selon l'invention, Lys-Ala (SEQ ID NO: 1), procure déjà un effet significatif d'inhibition de la tyrosinase même à une concentration aussi faible que $5.10^{-6}$ M, cet effet d'inhibition étant sensiblement amélioré aux faibles concentrations après un certain temps de contact du peptide inhibiteur avec la tyrosinase, avant mise en contact avec le substrat. Cet effet est grandement amplifié en augmentant la concentration en peptide inhibiteur.

Ainsi, le peptide Lys-Ala (SEQ ID NO: 1) présente une très forte activité inhibitrice de la tyrosinase, ce qui entraîne une forte activité dépigmentante par diminution ou suppression de la formation de mélanine.

Exemple 2

Détermination de l'activité dépigmentante de l'hexapeptide Lys-Cys-Thr-Cys-Cys-Ala (SEQ ID NO: 2)

On procède comme décrit à l'exemple 1, si ce n'est qu'on utilise le présent hexapeptide en lieu et place dudit peptide Lys-Ala, à diverses concentrations.

Les résultats sont répertoriés au tableau II ainsi qu'à la figure 2 sous forme d'histogramme de manière similaire à la figure 1.

TABLEAU II

| Conc. hexapeptide / Temps | $5.10^{-6}$ M | $5.10^{-5}$ M | $5.10^{-4}$ M | $1.10^{-3}$ M | $5.10^{-3}$ M |
|---|---|---|---|---|---|
| T 0 | 6 | 8 | 14 | 70 | 98 |
| T 5 min | 8 | 11 | 40 | 96 | 100 |
| T 15 min | 15 | 22 | 98 | 100 | 100 |

Il résulte du tableau II ainsi que de la figure 2 annexée que l'activité inhibitrice de l'hexapeptide (Lys-Cys-Thr-Cys-Cys-Ala) (SEQ ID NO: 2) de l'invention est déjà très significative à la concentration de $5.10^{-6}$ M. Cette activité inhibitrice est radicalement augmentée lorsque la mise en contact avec le substrat est précédée par un temps de contact relativement prolongé du peptide de l'invention avec la tyrosinase, ou si l'on augmente la concentration en peptide inhibiteur. Ces résultats vont dans le même sens que ceux de l'exemple 1. En outre, l'inhibition de la tyrosinase conduisant à un excellent effet dépigmentant, est presque de 100 % (98 %) dès la concentration de $5.10^{-4}$ M dans la série d'essais à T 15 min. A une concentration plus élevée, par exemple à $5.10^{-3}$ M, l'inhibition quasi complète (98 %) apparaît déjà dans la série d'essais $T_0$, ce qui est tout à fait remarquable.

Il résulte ainsi des essais précédents, réalisés avec les peptides des exemples 1 et 2, la mise en évidence d'une forte activité dépigmentante pour des peptides à terminaison Lys et Ala, ce qui constitue une nouvelle famille de substances dépigmentantes.

Ainsi, l'invention trouve des applications particulièrement intéressantes dans les compositions cosmétiques ou pharmaceutiques, notamment dermatologiques à activité dépigmentante. Des exemples de formulations de telles compositions cosmétiques ou pharmaceutiques à activité dépigmentante sont donnés ci-après.

Exemple 3

Crème anti-tache de sénescence

| | |
|---|---|
| - hexapeptide (Lys-Cys-Thr-Cys-Cys-Ala) (SEQ ID NO: 2) | 1,00 g |
| - excipient émulsionné parfumé | q.s.p. 100,00 g |

On mélange l'hexapeptide dans l'excipient émulsionné parfumé en obtenant ainsi une crème qui peut être appliquée au moins une fois par jour sur les taches de sénescence généralement localisées sur le visage et les mains ainsi que sur les zones exposées telles que les décolletés.

Exemple 4

Lotion capillaire éclaircissante

| | |
|---|---|
| - dipeptide Lys-Ala (SEQ ID NO: 1) | 0,1 g |
| - Carbopol 940® | 0,2 g |
| - excipient alcoolique à 60° parfumé | q.s.p. 100,00 g |

On mélange tout d'abord le dipeptide Lys-Ala dans un excipient alcoolique, puis on ajoute le Carbopol en obtenant ainsi une lotion capillaire qui peut être appliquée sur le cuir chevelu pendant plusieurs jours jusqu'à obtenir un effet éclaircissant.

Exemple 5

Composition cosmétique dépigmentante ou blanchissante

- liposome contenant le dipeptide et l'hexapeptide de l'invention, de formule pondérale :

| | |
|---|---|
| - phospholipide | 1 g |
| - dipeptide, Lys-Ala (SEQ ID NO: 1) | 0,1 g |
| - hexapeptide, Lys-Cys-Thr-Cys-Cys-Ala (SEQ ID NO: 2) | 3,0 g |
| - eau | q.s.p. 50,0 g |
| - excipient gélifié Carbopol 940® | q.s.p. 100 g |

On prépare cette composition de la manière suivante :

Tout d'abord, selon la méthode décrite dans le document US-A-4 508 703, on prépare une poudre lipidique avec les phospholipides puis on disperse cette poudre lipidique dans une solution aqueuse contenant 0,2 % en poids de dipeptide et 6 % en poids d'hexapeptide sous agitation, de manière à former une suspension de liposomes contenant le dipeptide et l'hexapeptide. Cette suspension est avantageusement homogénéisée soit au moyen d'ultrasons, soit selon le procédé décrit dans le document US-A-4 621 023.

On prend 50 g de cette suspension aqueuse homogénéisée de liposomes, à laquelle on ajoute 50 g d'excipient gélifié pour obtenir les 100 g de la composition recherchée sous forme gélifiée.

Cette composition peut être appliquée sur les taches de sénescence, par exemple du visage, pour obtenir l'effet dépigmentant ou blanchissant recherché.

LISTE DE SEQUENCES

(1) INFORMATION GENERALE:

(i) DEPOSANT:
(A) NOM: PARFUMS CHRISTIAN DIOR
(B) RUE: 33, avenue Hoche
(C) VILLE: PARIS
(E) PAYS: FRANCE
(F) CODE POSTAL: 75008
(G) TELEPHONE: 49.53.85.00
(H) TELECOPIE: 49.53.85.01

(ii) TITRE DE L' INVENTION: Utilisation d'un peptide ayant un groupement lysine et un groupement alanine en position terminale pour la preparation d'une composition depigmentante et composition depigmentante en comportant application

(iii) NOMBRE DE SEQUENCES: 2

(iv) FORME LISIBLE PAR ORDINATEUR:
(A) TYPE DE SUPPORT: Floppy disk
(B) ORDINATEUR: IBM PC compatible
(C) SYSTEME D' EXPLOITATION: PC-DOS/MS-DOS
(D) LOGICIEL: PatentIn Release #1.0, Version #1.25 (OEB)

(vi) DONNEES DE LA DEMANDE ANTERIEURE:
(A) NUMERO DE DEPOT: FR 9307319
(B) DATE DE DEPOT: 17-JUN-1993

(2) INFORMATION POUR LA SEQ ID NO: 1:

(i) CARACTERISTIQUES DE LA SEQUENCE:
(A) LONGUEUR: 2 acides aminés
(B) TYPE: acide aminé
(C) NOMBRE DE BRINS: simple
(D) CONFIGURATION: linéaire

(ii) TYPE DE MOLECULE: peptide

(iii) HYPOTHETIQUE: NON

```
(xi) DESCRIPTION DE LA SEQUENCE: SEQ ID NO: 1:

 Lys Ala
 1

(2) INFORMATION POUR LA SEQ ID NO: 2:

  (i) CARACTERISTIQUES DE LA SEQUENCE:
       (A) LONGUEUR: 6 acides aminés
       (B) TYPE: acide aminé
       (C) NOMBRE DE BRINS: simple
       (D) CONFIGURATION: linéaire

 (ii) TYPE DE MOLECULE: peptide

(iii) HYPOTHETIQUE: NON

 (xi) DESCRIPTION DE LA SEQUENCE: SEQ ID NO: 2:

 Lys Cys Thr Cys Cys Ala
 1               5
```

## Revendications

1. Utilisation d'un peptide constitué d'une chaîne peptidique comportant à l'une de ses deux extrémités un groupement lysine (Lys), et à son autre extrémité un groupement alanine (Ala), pour la préparation d'une composition dépigmentante, destinée en particulier à atténuer ou à supprimer les taches mélaniques de la peau, telles que les taches dites de sénescence, ou à éclaircir la chevelure.

2. Utilisation selon la revendication 1, caractérisée en ce que le nombre d'acides aminés formant ladite chaîne peptidique est compris entre 2 et 10 inclus.

3. Utilisation selon la revendication 1 ou 2, caractérisée en ce que le groupement lysine précité est situé en position dite N-terminale, et le groupement alanine précité situé en position dite C-terminale.

4. Utilisation selon l'une des revendications 1 à 3, caractérisée en ce que le peptide précité est le dipeptide Lys-Ala (SEQ ID NO: 1).

5. Utilisation selon l'une des revendications 1 à 3, caractérisée en ce que le peptide précité est l'hexapeptide de formule :

   Lys-Cys-Thr-Cys-Cys-Ala (SEQ ID NO: 2).

6. Utilisation selon l'une des revendications 1 à 5, caractérisée en ce que le peptide précité est utilisé à une concentration comprise entre 0,01 et 5 % en poids.

7. Utilisation selon l'une des revendications 1 à 6, caractérisée en ce que le peptide précédemment défini est incorporé au moins en partie dans des vecteurs de substance active tels qu'une phase lamellaire lipidique hydratée ou des liposomes.

8. Composition cosmétique ou pharmaceutique, notamment dermatologique, à activité dépigmentante, caractérisée en ce qu'elle comprend un peptide constitué d'une chaîne peptidique comportant à l'une de ses deux extrémités un groupement lysine (Lys), et à son autre extrémité un groupement alanine (Ala), ledit peptide étant incorporé dans un excipient cosmétiquement ou pharmaceutiquement acceptable.

9. Composition selon la revendication 8, caractérisée en ce que le nombre d'acides aminés formant ladite chaîne peptidique est compris entre 2 et 10 inclus.

10. Composition selon la revendication 8 ou 9, caractérisée en ce que le groupement lysine précité est situé en position dite N-terminale, et le groupement alanine précité situé en position dite C-terminale.

11. Composition selon l'une des revendication 8 à 10, caractérisée en ce que le peptide précité est le dipeptide Lys-Ala (SEQ ID NO: 1).

12. Composition selon l'une des revendications 8 à 11, caractérisée en ce que le peptide précité est l'hexapeptide de formule :
    Lys-Cys-Thr-Cys-Cys-Ala (SEQ ID NO: 2).

13. Composition selon l'une des revendications 8 à 12, caractérisée en ce que le peptide précité est utilisé à une concentration comprise entre 0,01 et 5 % en poids.

14. Composition selon l'une des revendications 8 à 13, caractérisée en ce que le peptide précédemment défini est incorporé au moins en partie dans des vecteurs de substance active tels qu'une phase lamellaire lipidique hydratée ou des liposomes.

15. Procédé cosmétique de dépigmentation de la peau ou des cheveux, caractérisé en ce qu'il comprend l'application sur les zones de la peau devant être dépigmentées ou sur le cuir chevelu, d'une quantité efficace d'un peptide tel que défini à l'une quelconque des revendications 1 à 7, incorporée dans une composition cosmétique.

**Claims**

1. The use of a peptide constituted by a peptide chain containing a lysine (Lys) group at one of its two ends and an alanine (Ala) group at its other end, for the preparation of a depigmenting composition, aimed particularly for attenuating or removing melanistic spots on the skin such as age spots, or for lightening the hair.

2. Use according to claim 1, characterized in that the number of amino acids forming said peptide chain is in the range 2 to 10 inclusive.

3. Use according to claim 1 or claim 2, characterized in that said lysine group is located at the N-terminal position and said alanine group is located at the C-terminal position.

4. Use according to any one of claims 1 to 3, characterized in that said peptide is the dipeptide Lys-Ala (SEQ ID NO:1).

5. Use according to any one of claims 1 to 3, characterized in that said peptide is the hexapeptide with formula:
    Lys-Cys-Thr-Cys-Cys-Ala (SEQ ID NO:2).

6. Use according to any one of claims 1 to 5, characterized in that said peptide is used at a concentration in the range 0.01% and 5% by weight.

7. Use according to any one of claims 1 to 6, characterized in that said peptide is at least partially incorporated into active ingredient vectors such as a hydrated lamellar lipidic phase or into liposomes.

8. A cosmetic or pharmaceutical composition, in particular a dermatological composition, with a depigmenting activity, characterized in that it comprises a peptide constituted by a peptide chain containing a lysine (Lys) group at one of its two ends and an alanine (Ala) group at its other end, said peptide being incorporated into a cosmetically or pharmaceutically acceptable excipient.

9. A composition according to claim 8, characterized in that the number of amino acids forming said peptide chain is in the range 2 to 10 inclusive.

10. A composition according to claim 8 or claim 9, characterized in that said lysine group is located at the N-terminal position and said alanine group is located at the C-terminal position.

11. A composition according to any one of claims 8 to 10, characterized in that said peptide is the dipeptide Lys-Ala (SEQ ID NO:1).

12. A composition according to any one of claims 8 to 11, characterized in that said peptide is the hexapeptide with formula:

    Lys-Cys-Thr-Cys-Cys-Ala (SEQ ID NO:2).

13. A composition according to any one of claims 8 to 12, characterized in that said peptide is used at a concentration in the range 0.01% and 5% by weight.

14. A composition according to any one of claims 8 to 13, characterized in that said peptide is at least partially incorporated into active ingredient vectors such as a hydrated lamellar lipidic phase or into liposomes.

15. A method for the cosmetic depigmenting of skin or hair, characterized in that it comprises applying to zones of the skin to be depigmented or to the scalp, an effective quantity of a peptide as defined in any one of claims 1 to 7, incorporated in a cosmetic composition.

**Patentansprüche**

1. Verwendung eines Peptids, das aus einer Peptidkette besteht, die an einem ihrer Enden eine Lysin (Lys)-Gruppe und an ihrem anderen Ende eine Alanin (Ala)-Gruppe umfaßt, bei der Herstellung einer depigmentierenden Zusammensetzung, die insbesondere zur Abschwächung oder Unterdrückung von Melaninflecken der Haut, wie Altersflecken, oder zur Aufhellung der Haare bestimmt ist.

2. Verwendung nach Anspruch 1, dadurch gekennzeichnet, daß die Anzahl von die Peptidkette bildenden Aminosäuren zwischen 2 und 10 einschließlich beträgt.

3. Verwendung nach Anspruch 1 oder 2, dadurch gekennzeichnet, daß die Lysin-Gruppe in der N-terminalen Stellung vorliegt, und die Alanin-Gruppe in der C-terminalen Stellung vorliegt.

4. Verwendung nach einem der Ansprüche 1 bis 3, dadurch gekennzeichnet, daß das Peptid das Dipeptid Lys-Ala (SEQ ID NO: 1) ist.

5. Verwendung nach einem der Ansprüche 1 bis 3, dadurch gekennzeichnet, daß das Peptid das Hexapeptid der Formel:

    Lys-Cys-Thr-Cys-Cys-Ala (SEQ ID NO: 2)

   ist.

6. Verwendung nach einem der Ansprüche 1 bis 5, dadurch gekennzeichnet, daß das Peptid bei einer Konzentration zwischen 0,01 und 5 Masse-% verwendet wird.

7. Verwendung nach einem der Ansprüche 1 bis 6, dadurch gekennzeichnet, daß das vorstehend definierte Peptid zumindest teilweise in Vektoren einer aktiven Substanz, wie einer hydratisierten lamellaren Lipidphase oder Liposomen, eingeschlossen ist.

8. Kosmetische oder pharmazeutische, insbesondere dermatologische Zusammensetzung mit depigmentierender Wirksamkeit, dadurch gekennzeichnet, daß sie ein Peptid enthält, das aus einer Peptidkette besteht, die an einem ihrer Enden eine Lysin (Lys)-Gruppe und an ihrem anderen Ende eine Alanin (Ala)-Gruppe umfaßt, wobei das Peptid in einem kosmetisch oder pharmazeutisch annehmbaren Exzipienten eingeschlossen ist.

9. Zusammensetzung nach Anspruch 8, dadurch gekennzeichnet, daß die Anzahl von die Peptidkette bildenden Aminosäuren zwischen 2 und 10 einschließlich beträgt.

**10.** Zusammensetzung nach Anspruch 8 oder 9, dadurch gekennzeichnet, daß die Lysin-Gruppe in der N-terminalen Stellung vorliegt, und die Alanin-Gruppe in der C-terminalen Stellung vorliegt.

**11.** Zusammensetzung nach einem der Ansprüche 8 bis 10, dadurch gekennzeichnet, daß das Peptid das Dipeptid Lys-Ala (SEQ ID NO: 1) ist.

**12.** Zusammensetzung nach einem der Ansprüche 8 bis 11, dadurch gekennzeichnet, daß das Peptid das Hexapeptid der Formel:

Lys-Cys-Thr-Cys-Cys-Ala (SEQ ID NO: 2)

ist.

**13.** Zusammensetzung nach einem der Ansprüche 8 bis 12, dadurch gekennzeichnet, daß das Peptid bei einer Konzentration zwischen 0,01 und 5 Masse-% verwendet wird.

**14.** Zusammensetzung nach einem der Ansprüche 8 bis 13, dadurch gekennzeichnet, daß das vorstehend definierte Peptid zumindest teilweise in Vektoren einer aktiven Substanz, wie einer hydratisierten lamellaren Lipidphase oder Liposomen, eingeschlossen ist.

**15.** Kosmetisches Verfahren zur Depigmentierung der Haut oder der Haare, dadurch gekennzeichnet, daß es das Aufbringen einer wirksamen Menge eines Peptids, wie in einem der Ansprüche 1 bis 7 definiert, die in eine kosmetische Zusammensetzung eingeschlossen wird, auf zu depigmentiere Zonen der Haut oder auf die Kopfhaut umfaßt.

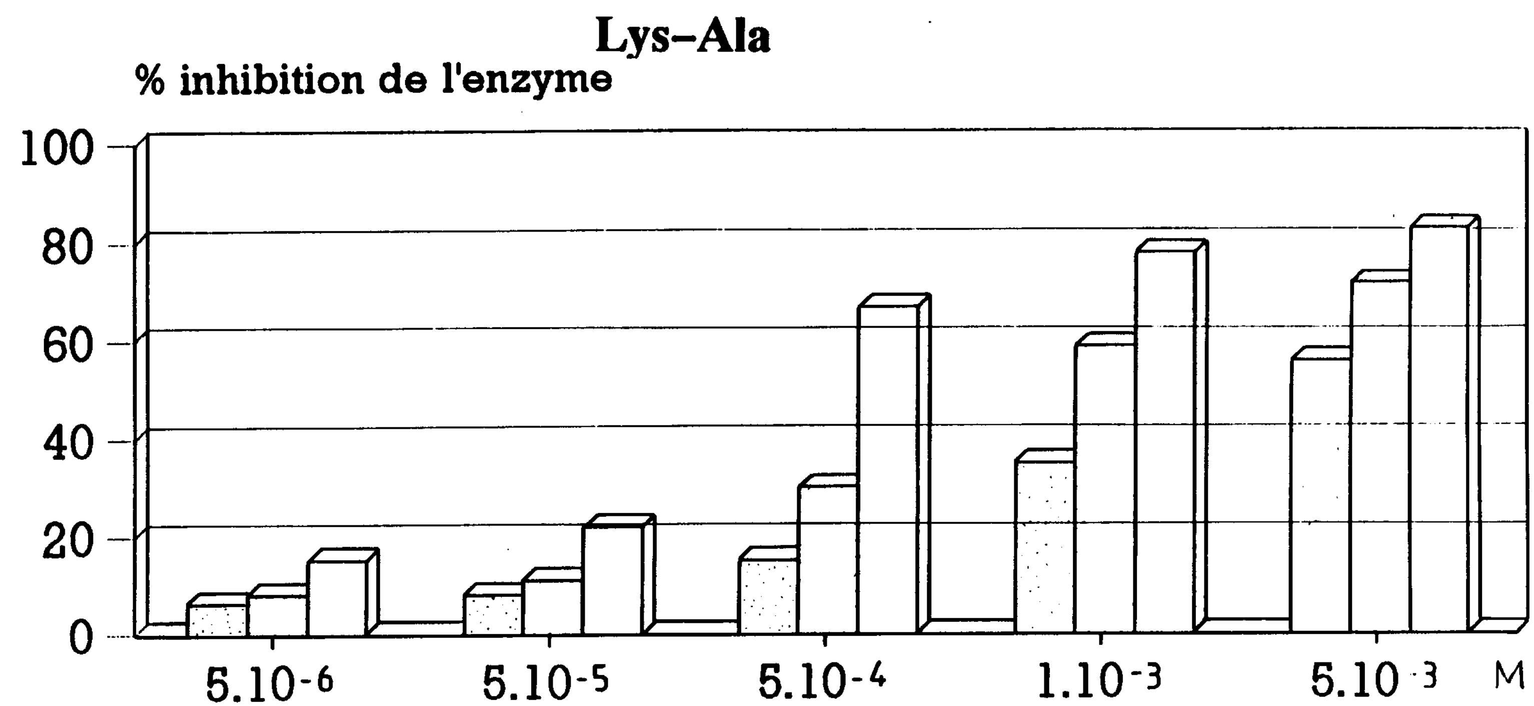


| | | | | | |
|---|---|---|---|---|---|
| T 0 | 6 | 8 | 15 | 35 | 56 |
| T 5 min. | 8 | 11 | 30 | 59 | 72 |
| T 15 min. | 15 | 22 | 67 | 78 | 83 |

temps incubation

T 0 · T 5 min. · T 15 min.

FIG.1

**Lys-Cys-Thr-Cys-Cys-Ala**

Activité dépigmentante de l'hexapeptide selon l'invention

**% inhibition de l'enzyme**

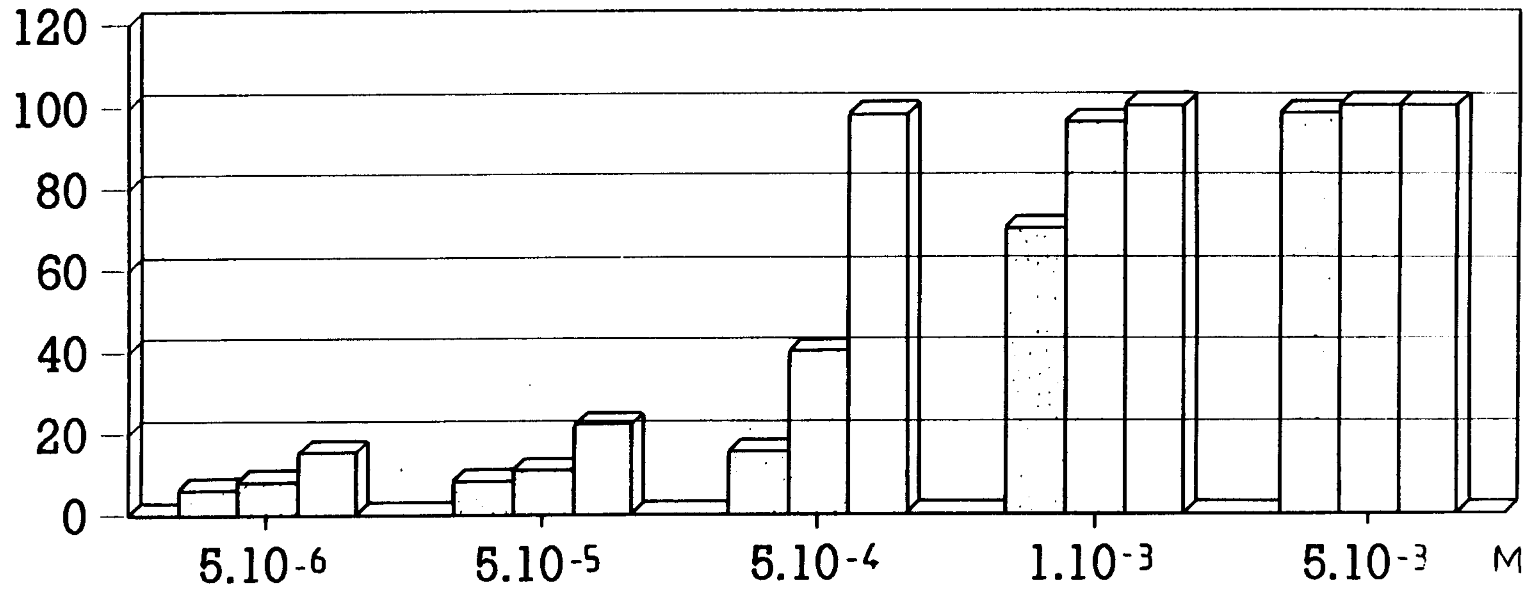


| | | | | | |
|---|---|---|---|---|---|
| T 0 | 6 | 8 | 15 | 70 | 98 |
| T 5 min. | 8 | 11 | 40 | 96 | 100 |
| T 15 min. | 15 | 22 | 98 | 100 | 100 |

temps incubation

T 0 T 5 min. T 15 min.

FIG.2